(19) Europäisches Patentamt
European Patent Office
Office européen des brevets

(11) Publication number: 0 450 102 A1

(12) EUROPEAN PATENT APPLICATION
published in accordance with Art. 158(3) EPC

(21) Application number: 90915826.3

(22) Date of filing: 25.10.90

(86) International application number: PCT/JP90/01371

(87) International publication number: WO 91/06555 (16.05.91 91/11)

(51) Int. Cl.$^5$: C07H 19/16, C07H 19/23, A61K 31/70

(30) Priority: 26.10.89 JP 279437/89

(43) Date of publication of application: 09.10.91 Bulletin 91/41

(84) Designated Contracting States: CH DE ES FR GB GR IT LI

(71) Applicant: Yamasa Shoyu Kabushiki Kaisha
10-1, Araoi-Cho 2-Chome
Choshi-Shi Chiba-Ken 288(JP)

(72) Inventor: MATSUDA, Akira, 10-501, Minamishinkawa Koumin Shukusha, Monbusho-Youchi, Nishi 13-chome
Kita 23 Jo, Kita-ku,Sapporo-shi,Hokkaido(JP)
Inventor: WATAYA, Yusuke
2-2-58-102, Kadotayashiki
Okayama-shi, Okayama 703(JP)
Inventor: ISHII, Akira
2-22-4, Kunitomi
Okayama-shi, Okayama 703(JP)
Inventor: UEDA, Tohru

deceased(JP)

(74) Representative: Dr. Elisabeth Jung Dr. Jürgen Schirdewahn Dipl.-Ing. Claus Gernhardt
P.O. Box 40 14 68 Clemensstrasse 30
W-8000 München 40(DE)

# (54) NUCLEOSIDE DERIVATIVE.

(57) Nucleoside derivatives having a potent antiprotozoan activity and a reduced adverse effect and being effective in treating diseases caused by protozoan, such as 2'- and/or 5'-acylated, aralkylated or silylated 3'-deoxyinosine; allopurinol 3'-deoxyriboside; 2'- and/or 5'-acylated, aralkylated or silylated allopurinol 3'-deoxyriboside; and 2'-, 3'-, and/or 5'-acylated, aralkylated or silylated allopurinol riboside.

EP 0 450 102 A1

## TECHNICAL FIELD

The present invention relates to novel nucleoside derivatives and an antiprotozoal agent containing the nucleoside derivatives as an effective ingredient.

## BACKGROUND ART

Trypanosomiasis (sleeping sickness and Chagas' disease) caused by parasitic protozoa belonging to Trypanosoma genus and leishmaniasis (e.g., Kala-azar, oriental sore and American leishmaniasis) caused by parasitic protozoa belonging to Leishmania genus are known as the infectious diseases caused by parasitic protozoa, which previal mainly in the tropical and subtropical zones.

In the past, these infectious diseases caused by parasitic protozoa have been treated by using pentostam, suramin, antimony compound, diamidine derivatives and the like, but all these drugs are not necessarily satisfactory due to their strong side effects (e.g., toxicity).

The present inventors have already reported that inosine analogs such as 3'-deoxyinosine and carbocyclic inosine are effective on the above infectious diseases with such side effects that are not so strong as that of the drugs used previously [See, e.g., Japan Tropic Medicine Institute Bulletin, 16, No. 4, pp. 317 - 325 (1988)].

The antiprotozoal effect of inosine analogs such as allopurinol riboside, 7-deazainosine, 9-deazainosine, formycin B, 8-azainosine and 7-thio-7,9-dideazainosine have been also reported, e.g., in Antimicrob. Agents Chemother., 25, 292 (1984), and ibid., 27, 33 (1985).

However, the above inosine analogs fail to have sufficient main effect, i.e., antiprotozoal effect. Thus, there is an earnest desire for the development of the drugs having stronger main effect with the side effects which are as weak as that of the above inosine analogs, and showing such effect by oral administration as well.

## DISCLOSURE OF THE INVENTION

As a result of continued various researches to achieve enhancement of the main effect of inosine analogs, the present inventors have found that the nucleoside derivatives represented by the following formula [I] and the pharmaceutically acceptable salts thereof have a potent antiprotozoal effect, and have accomplished the present invention.

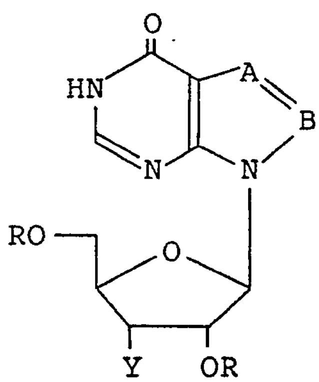

[I]

[wherein A and B are different, and are each a carbon atom or a nitrogen atom, Y is a hydrogen atom, a hydroxyl group or -OR, and R's are the same or different, and are each a hydrogen atom, an aralkyl group, a silyl group or an acyl group, with the exception of the compounds represented by the following combinations;

(1) A is a nitrogen atom, B is a carbon atom, Y is a hydroxyl group or -OR, and R's are the same or different, and are each a hydrogen atom, an aralkyl group, a silyl group or an acyl group (inosine and an aralkylated, a silylated and an acylated of inosine),

(2) A is a nitrogen atom, B is a carbon atom, Y is a hydrogen atom, and all R's are hydrogen atoms simultaneously (3'-deoxyinosine).

(3) A is a carbon atom, B is a nitrogen atom, Y is a hydroxyl group, and all R's are hydrogen atoms simultaneously (allopurinol riboside)].

The present invention relates to the nucleoside derivatives represented by the Formula [I] as mentioned above or the pharmaceutically acceptable salts thereof and the antiprotozoal agent which comprises containing as an effective ingredient them.

BRIEF DESCRIPTION OF THE DRAWING

Fig. 1 is a graph indicating the therapeutical effect of the test compounds on mice infected with Leishmania donovani.

Fig. 2 is a graph illustrating changes of the body weights during two weeks after Leishmania donovani inoculation.

Fig. 3 is a graph indicating weight of the spleen and the liver.

Fig. 4 is a graph indicating the hematocrit and plasma protein content.

BEST MODE FOR CARRYING OUT THE INVENTION

[I] The Compounds of the Present Invention

The compounds of the present invention are represented by the above Formula [I]. In the formula, the acyl groups for R's are the same or different, and are each represented by the formula $R^1CO$- (wherein $R^1$ is an alkyl group, an aralkyl group or an aryl group).

The alkyl group herein refers to a straight or branched chain alkyl group having one to 19 carbon atoms such as, for example, methyl, ethyl, propyl, isopropyl, butyl, isobutyl, sec-butyl, tert-butyl, pentyl, isopentyl, neopentyl, hexyl, isohexyl, heptyl, octyl, nonyl, decyl, undecyl, dodecyl, tridecyl, tetradecyl, pentadecyl, hexadecyl, heptadecyl and nonadecyl.

Among the preferred alkyl group are those having not less than 5 carbon atoms.

The aralkyl group refers to an unsubstituted or substituted aralkyl group such as, for example, benzyl, 1-phenylethyl, methylbenzyl, halobenzyl (e.g., fluorobenzyl and chlorobenzyl), methoxybenzyl, dimethoxybenzyl, nitrobenzyl, phenethyl, picolyl and 3-indolylmethyl.

The aryl group refers to an unsubstituted or substituted aryl group such as, for example, phenyl, tolyl, xylyl, mesityl, cumenyl, ethylphenyl, halophenyl (e.g., fluorophenyl, chlorophenyl, iodophenyl, bromophenyl, difluorophenyl and dichlorophenyl), alkoxyphenyl (e.g., methoxyphenyl, ethoxyphenyl and dimethoxyphenyl), methylenedioxyphenyl, nitrophenyl, cyanophenyl, carbamoylphenyl, methoxycarbonylphenyl, naphthyl, biphenyl, thienyl, furyl, pyrrolyl, imidazolyl, pyrazolyl, pyridyl and pyrazinyl.

The aralkyl groups for R's in Formula [I] are the same or different, and are the same as those as illustrated for $R^1$.

The silyl groups for R's in Formula [I] are the same or different, and are each an alkylsilyl group such as, for example, trimethylsilyl, triethylsilyl, methyldiisopropylsilyl, triisopropylsilyl, tert-butyldimethylsilyl, methyl-di-tert-butylsilyl and thexyldimethylsilyl.

The compound of the present invention also includes the pharmaceutically acceptable salts of the nucleoside derivatives of Formula [I]. The salts are metal salts such as sodium salts, potassium salts and calcium salts.

The compounds of the present invention of Formula [I] may be a keto-form

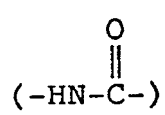

or an enol-form

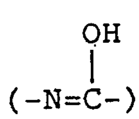

in the chemical structure of the base portion in the compound.

Typical compounds of the present invention are illustrated by 3'-deoxyinosine derivatives represented by Formula [I']

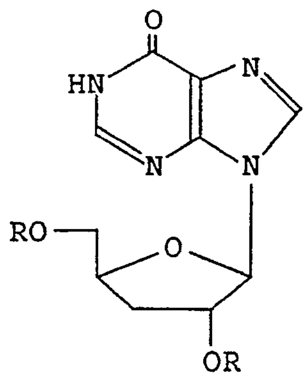

[I']

(wherein R's are the same or different, and are each a hydrogen atom, an aralkyl group, a silyl group or an acyl group, with the exception of the compound in which all R's are hydrogen atoms simultaneously).

The compounds of Formula [I'] are illustrated more specifically as follows:

(1) 5'-O-Aralkyl-3'-deoxyinosine
5'-O-Benzyl-3'-deoxyinosine;
5'-O-Halobenzyl-3'-deoxyinosine;
5'-O-Methoxybenzyl-3'-deoxyinosine, etc.

(2) 2'-O-Aralkyl-3'-deoxyinosine
2'-O-Benzyl-3'-deoxyinosine;
2'-O-Halobenzyl-3'-deoxyinosine;
2'-O-Methoxybenzyl-3'-deoxyinosine, etc.

(3) 2',5'-Di-O-aralkyl-3'-deoxyinosine
2',5'-Di-O-benzyl-3'-deoxyinosine;
2',5'-Di-O-halobenzyl-3'-deoxyinosine;
2',5'-Di-O-methoxybenzyl-3'-deoxyinosine, etc.

(4) 5'-O-Silyl-3'-deoxyinosine
5'-O-tert-Butyldimethylsilyl-3'-deoxyinosine;
5'-O-Thexyldimethylsilyl-3'-deoxyinosine;
5'-O-Trimethylsilyl-3'-deoxyinosine, etc.

(5) 2'-O-Silyl-3'-deoxyinosine
2'-O-tert-Butyldimethylsilyl-3'-deoxyinosine;
2'-O-Thexyldimethylsilyl-3'-deoxyinosine;
2'-O-Trimethylsilyl-3'-deoxyinosine, etc.

(6) 2',5'-Di-O-silyl-3'-deoxyinosine
2',5'-Di-O-tert-butyldimethylsilyl-3'-deoxyinosine;
2',5'-Di-O-thexyldimethylsilyl-3'-deoxyinosine;
2',5'-Di-O-trimethylsilyl-3'-deoxyinosine, etc.

(7) 5'-O-Acyl-3'-deoxyinosine
5'-O-Stearoyl-3'-deoxyinosine;
5'-O-Palmitoyl-3'-deoxyinosine;
5'-O-Myristoyl-3'-deoxyinosine;
5'-O-Decanoyl-3'-deoxyinosine, etc.

(8) 2'-O-Acyl-3'-deoxyinosine
2'-O-Stearoyl-3'-deoxyinosine;
2'-O-Palmitoyl-3'-deoxyinosine;
2'-O-Myristoyl-3'-deoxyinosine;
2'-O-Decanoyl-3'-deoxyinosine, etc.

(9) 2',5'-Di-O-acyl-3'-deoxyinosine
2',5'-Di-O-stearoyl-3'-deoxyinosine;
2',5'-Di-O-palmitoyl-3'-deoxyinosine;
2',5'-Di-O-decanoyl-3'-deoxyinosine;
2'-O-Decanoyl-5'-O-stearoyl-3'-deoxyinosine, etc.

Another embodiments of the compound of the present invention are allopurinol riboside derivatives, allopurinol-3'-deoxyriboside and derivatives thereof, all of which are represented by Formula [I"]

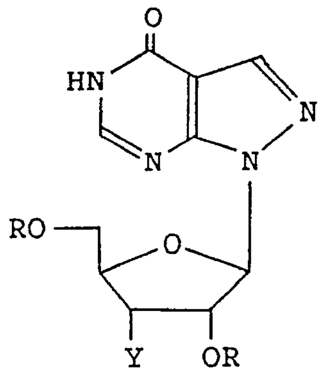

[I"]

(wherein Y is a hydrogen atom, a hydroxyl group or -OR, and R's are as defined above, with the exception of the compound in which all R's are hydrogen atoms simultaneously when Y is a hydroxyl group).

The compounds of Formula [I"] are illustrated more specifically as follows:

(1) 1-(β-D-3-Deoxyribofuranosyl)allopurinol [Allopurinol-3'-deoxylriboside],

(2) 1-(β-D-5-O-Aralkyl-3-deoxyribofuranosyl)allopurinol
1-(β-D-5-O-Benzyl-3-deoxyribofuranosyl)allopurinol
1-(β-D-5-O-Halobenzyl-3-deoxyribofuranosyl)allopurinol,etc.

(3) 1-(β-D-2-O-Aralkyl-3-deoxyribofuranosyl)allopurinol
1-(β-D-2-O-Benzyl-3-deoxyribofuranosyl)allopurinol
1-(β-D-2-O-Halobenzyl-3-deoxyribofuranosyl)allopurinol, etc.

(4) 1-(β-D-2,5-Di-O-aralkyl-3-deoxyribofuranosyl)allopurinol
1-(β-D-2,5-Di-O-benzyl-3-deoxyribofuranosyl)allopurinol;
1-(β-D-2,5-Di-O-halobenzyl-3-deoxyribofuranosyl)allopurinol, etc.

(5) 1-(β-D-5-O-Silyl-3-deoxyribofuranosyl)allopurinol
1-(β-D-5-O-tert-Butyldimethylsilyl-3-deoxyribofuranosyl)allopurinol;
1-(β-D-5-O-Thexyldimethylsilyl-3-deoxyribofuranosyl)allopurinol, etc.

(6) 1-(β-D-2-O-Silyl-3-deoxyribofuranosyl)allopurinol
1-(β-D-2-O-tert-Butyldimethylsilyl-3-deoxyribofuranosyl)allopurinol;
1-(β-D-2-O-Thexyldimethylsilyl-3-deoxyribofuranosyl)allopurinol, etc.

(7) 1-(β-D-2,5-Di-O-silyl-3-deoxyribofuranosyl)allopurinol
1-(β-D-2,5-Di-O-tert-butyldimethylsilyl-3-deoxyribofuranosyl)allopurinol;
1-(β-D-2,5-Di-O-thexyldimethylsilyl-3-deoxyribofuranosyl)allopurinol, etc.

(8) 1-(β-D-5-O-Acyl-3-deoxyribofuranosyl)allopurinol
1-(β-D-5-O-Stearoyl-3-deoxyribofuranosyl)allopurinol;
1-(β-D-5-O-Palmitoyl-3-deoxyribofuranosyl)allopurinol;
1-(β-D-5-O-Decanoyl-3-deoxyribofuranosyl)allopurinol, etc.

(9) 1-(β-D-2-O-Acyl-3-deoxyribofuranosyl)allopurinol
1-(β-D-2-O-Stearoyl-3-deoxyribofuranosyl)allopurinol;
1-(β-D-2-O-Palmitoyl-3-deoxyribofuranosyl)allopurinol;
1-(β-D-2-O-Decanoyl-3-deoxyribofuranosyl)allopurinol, etc.

(10) 1-(β-D-2,5-Di-O-acyl-3-deoxyribofuranosyl)allopurinol
1-(β-D-2,5-Di-O-stearoyl-3-deoxyribofuranosyl)allopurinol;
1-(β-D-2,5-Di-O-palmitoyl-3-deoxyribofuranosyl)allopurinol, etc.

(11) 1-(β-D-5-O-Aralkylribofuranosyl)allopurinol
1-(β-D-5-O-Benzylribofuranosyl)allopurinol;
1-(β-D-5-O-Methoxybenzylribofuranosyl)allopurinol, etc.

(12) 1-(β-D-2-O-Aralkylribofuranosyl)allopurinol
1-(β-D-2-O-Benzylribofuranosyl)allopurinol;
1-(β-D-2-O-Halobenzylribofuranosyl)allopurinol, etc.

(13) 1-(β-D-3-O-Aralkylribofuranosyl)allopurinol
1-(β-D-3-O-Benzylribofuranosyl)allopurinol;
1-(β-D-3-O-Halobenzylribofuranosyl)allopurinol, etc.

(14) 1-(β-D-2,3-Di-O-aralkylribofuranosyl)allopurinol
1-(β-D-2,3-Di-O-benzylribofuranosyl)allopurinol;

1-(β-D-2,3-Di-O-halobenzylribofuranosyl)allopurinol, etc.

(15) 1-(β-D-2,5-Di-O-aralkylribofuranosyl)allopurinol
1-(β-D-2,5-Di-O-benzylribofuranosyl)allopurinol;
1-(β-D-2,5-Di-O-halobenzylribofuranosyl)allopurinol, etc.

(16) 1-(β-D-3,5-Di-O-aralkylribofuranosyl)allopurinol
1-(β-D-3,5-Di-O-benzylribofuranosyl)allopurinol, etc.

(17) 1-(β-D-2,3,5-Tri-O-aralkylribofuranosyl)allopurinol
1-(β-D-2,3,5-Tri-D-benzylribofuranosyl)allopurinol;
1-(β-D-2,3,5-Tri-O-halobenzylribofuranosyl)allopurinol, etc.

(18) 1-(β-D-5-O-Silylribofuranosyl)allopurinol
1-(β-D-5-O-tert-Butyldimethylsilylribofuranosyl)allopurinol;
1-(β-D-5-O-Thexyldimethylsilylribofuranosyl)allopurinol, etc.

(19) 1-(β-D-2-O-Silylribofuranosyl)allopurinol
1-(β-D-2-O-tert-Butyldimethylsilylribofuranosyl)allopurinol;
1-(β-D-2-O-Thexyldimethylsilylribofuranosyl)allopurinol, etc.

(20) 1-(β-D-3-O-Silylribofuranosyl)allopurinol
1-(β-D-3-O-tert-Butyldimethylsilylribofuranosyl)allopurinol;
1-(β-D-3-O-Thexyldimethylsilylribofuranosyl)allopurinol, etc.

(21) 1-(β-D-2,3-Di-O-silylribofuranosyl)allopurinol
1-(β-D-2,3-Di-O-tert-butyldimethylsilylribofuranosyl)allopurinol, etc.

(22) 1-(β-D-2,5-Di-O-silylribofuranosyl)allopurinol
1-(β-D-2,5-Di-O-tert-butyldimethylsilylribofuranosyl)allopurinol, etc.

(23) 1-(β-D-3,5-Di-O-silylribofuranosyl)allopurinol
1-(β-D-3,5-Di-O-tert-butyldimethylsilylribofurnaosyl)allopurinol, etc.

(24) 1-(β-2,3,5-Tri-O-silylribofuranosyl)allopurinol
1-(β-D-2,3,5-Tri-O-tert-butyldimethylsilylribofuranosyl)allopurinol, etc.

(25) 1-(β-D-5-O-Acylribofuranosyl)allopurinol
1-(β-D-5-O-Stearoylribofurnaosyl)allopurinol;
1-(β-D-5-O-Palmitoylribofuranosyl)allopurinol;
1-(β-D-5-O-Decanoylribofuranosyl)allopurinol, etc.

(26) 1-(β-D-2-O-Acylribofuranosyl)allopurinol
1-(β-D-2-O-Stearoylribofuranosyl)allopurinol;
1-(β-D-2-O-Palmitoylribofuranosyl)allopurinol;
1-(β-n-2-o-Decanoylribofuranosyl)allopurinol, etc.

(27) 1-(β-D-3-O-Acylribofuranosyl)allopurinol
1-(β-D-3-O-Stearoylribofuranosyl)allopurinol;
1-(β-D-3-O-Myristoylribofuranosyl)allopurinol, etc.

(28) 1-(β-D-2,3-Di-O-acylribofuranosyl)allopurinol
1-(β-D-2,3-Di-O-stearoylribofuranosyl)allopurinol;
1-(β-D-2,3-Di-O-decanoylribofuranosyl)allopurinol, etc.

(29) 1-(β-D-2,5-Di-O-acylribofuranosyl)allopurinol
1-(β-D-2,5-Di-O-stearoylribofuranosyl)allopurinol;
1-(β-D-2,5-Di-O-myristoylribofuranosyl)allopurinol, etc.

(30) 1-(β-D-3,5-Di-O-acylribofuranosyl)allopurinol
1-(β-D-3,5-Di-O-stearoylribofuranosyl)allopurinol;
1-(β-D-3,5-Di-O-decanoylribofuranosyl)allopurinol, etc.

(31) 1-(β-D-2,3,5-Tri-O-acylribofuranosyl)allopurinol
1-(β-D-2,3,5-Tri-O-stearylribofuranosyl)allopurinol;
1-(β-D-2,3,5-Tri-O-decanoylribofuranosyl)allopurinol,etc.

[II] Process for Preparing the Compounds of the Present Invention

Of the compounds of the present invention, the compound having one or more members of aralkyl groups, silyl groups and acyl groups for R's can be prepared by using as a starting material a compound represented by the following Formula [II]

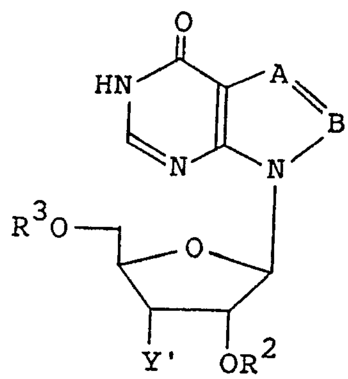

[II]

[wherein A and B are as defined above, Y' is a hydrogen atom or $-OR^4$ (wherein $R^4$ is a hydrogen atom or a protective group of a hydroxyl group), $R^2$ and $R^3$ are each a hydrogen atom or a protective group of a hydroxyl group, provided that $R^4$ together with $R^2$ or $R^3$ forms a cyclic protective group of hydroxyl groups when Y' is $-OR^4$] according to a method which comprises reacting the compound of Formula [II] with either a compound of Formula [III]

$XCOR^1$ [III]

(wherein X is a leaving group and $R^1$ is as defined above) or a compound of Formula [IV]

$HalR^5$ [IV]

(wherein Hal is a halogen atom, and $R^5$ is an aralkyl group or a silyl group), if desired, and further removing the protective groups of the hydroxyl groups.

Of the compounds of Formula [II],

3'-deoxyinosine (A is a nitrogen atom, B is a carbon atom, Y' is a hydrogen atom, $R^2$ and $R^3$ are each a hydrogen atom), and allopurinol riboside (A is a carbon atom, B is a nitrogen atom, Y' is a hydroxyl group, and $R^2$ and $R^3$ are each a hydrogen atom) are known compounds. Allopurinol-3'-deoxyriboside (A is a carbon atom, B is a nitrogen atom, Y' is a hydrogen atom, $R^2$ and $R^3$ are each a hydrogen atom) is a novel compound and can be prepared by a method similar to that of the preparation of allopurinol riboside [Tetrahedron Letters, 24, 2141 - 2144 (1977)] using 1-O-acetyl-2,5-di-O-benzoyl-β-D-3-deoxyribofuranose in place of 1-O-acetyl-2,3,5-tri-O-benzoyl-β-D-ribofuranose which is used in a condensation for preparing allopurinol riboside.

The protective group and the cyclic protective group of hydroxyl groups for $R^2$, $R^3$ and $R^4$ may be those used usually as protective groups of a hydroxyl group such as, for example, an acyl group (e.g., acetyl, propionyl, butylyl, benzoyl and naphthoyl), an acetal or ketal type protective group (ethylidene, propylidene, isopropylidene, benzylidene, cyclohexylidene, cyclopentylidene, methoxymethylidene, ethoxymethylidene and dimethoxymethylidene), an aralkyl group (e.g., benzyl, p-methoxybenzyl, 3,4-dimethoxybenzyl, diphenylmethyl, triphenylmethyl, α- or β-naphthylmethyl and α-naphthyldiphenylmethyl) and a silyl group (e.g., trimethylsilyl, tert-butyldimethylsilyl, methyldiisopropylsilyl, triisopropylsilyl and tetraisopropyldisiloxanyl).

Introduction of the protective group can be carried out according to usual manners. For example, introduction of a silyl group can be easily carried out by using 1 to 5 moles of a silylating agent (e.g., a silyl halide) relative to 1 mol of the compound to be silylated, in a reaction solvent (e.g., pyridine, picoline, diethylaniline, dimethylaminopyridine, dimethylformamide, acetonitrile, tetrabutylamine and triethylamine, respectively alone or in admixture) at 0 to 50 °C for 1 to 30 hours.

Example of the leaving group for X in the compound of Formula [III] to react with the compound of Formula [II] is a halogen atom, an acyloxy group, an acyloxycarbonyloxy group, a succinimidoxy group, a phthalimidoxy group, a 4-nitrophenyl group, an azido group or a 2,4,6-triisopropylbenzenesulfonyl group.

The aralkyl group and the silyl group for $R^5$ in the compound of Formula [IV] are the same as those mentioned above for R.

The reaction of the compound of Formula [II] with the compound of Formula [III] or [IV] can be easily carried out by using 1 to 10 moles of the compound of Formula [III] or [IV] relative to 1 mol of the compound of Formula [II] in a reaction solvent (e.g., pyridine, dioxane, tetrahydrofuran, acetonitrile, chloroform, dichloromethane, methanol, ethanol and water, respectively alone or in admixture) at 0 to

100°C.

The above reaction of the compound of Formula [II] with the compound of Formula [III] can be allowed to proceed more advantageously by adding a base (or acid acceptor) (e.g., triethylamine, pyridine, picoline, dimethylaminopyridine, lutidine, N,N-dimethylaniline, alkaline metal hydroxides, alkaline metal carbonates and alkaline metal phosphates) in the reaction solution.

The reaction of the compound of Formula [II] with the compound of Formula [IV] (wherein $R^5$ is an aralkyl group) can be allowed to proceed more advantageously by the addition of sodium hydride in the reaction solution.

The compounds of the present invention thus obtained, if necessary after removal of the protective groups of the hydroxyl groups, can be isolated and purified by suitable combination of usual manners such as chromatography, recrystallization, precipitation and extraction.

The compounds of the present invention exhibit antiprotozoal effect, therefore are useful as an antiprotozoal agent. For example, the compounds of the present invention have remarked growth inhibition activity against parasitic protozoa belonging to Trypanosomatidae trypanosoma and Trypanosomatidae leishmania when compared with known 3'-deoxyinosine, carbocylic inosine and allopurinol riboside.

The compounds of the present invention can be administered orally or parenterally to animals including human by various administration methods.

Oral administration forms may be solid pharmaceutical preparations (e.g., powders, granules capsules and tablets) or liquid pharmaceutical preparations (e.g., syrup and elixir). Parenteral administration forms may be injections, rectal administration forms, skin external preparations and inhalations. These pharmaceutical preparations can be formulated by adding any pharmaceutically acceptable adjuvants to the compound of the present invention according to a usual manner. Furthermore, sustained release preparations can be formulated according to known techniques.

For formulation of solid pharmaceutical preparations for oral administration, the compound of the present invention is mixed with fillers such as lactose, starch, crystalline cellulose, calcium lactate, calcium monohydrogen phosphate, magnesium aluminometasilicate and silicic acid anhydride to give powders. If necessary, the powders are mixed with binders (e.g., refined sugar, hydroxypropyl cellulose and polyvinylpyrrolidone) and disintegrators (e.g., carboxymethyl cellulose and carboxymethyl cellulose calcium), and subjected to wet or dry glanulation to give granules. For formulation of tablets, the above powers or granules can be punched into tablets as such or after mixing with lubricants (e.g., magnesium stearate and talc). Furthermore, the above powders or tablets are coated either with enteric bases (e.g., hydroxypropylmethyl cellulose phthalate and methyl methacrylate copolymer) to give enteric preparations, or with ethylcellulose, carnauba wax, hydrogenated oil and the like to give sustained release preparations. For formulation of capsules, the above powders or granules are filled into hard capsules, or the compound of the present invention is dissolved in glycerol, polyethylene glycol, sesame oil, olive oil and the like, and encapsulated with gelatin film to give soft capsules.

For formulation of liquid pharmaceutical preparations for oral administration, the compound of the present invention and sweeteners (e.g., refined sugar, sorbitol and glycerol) are dissolved in water to give a clear cyrup. The syrup can be further mixed either with essential oil, ethanol and the like to give an elixir, or with gum arabic, tragacanth gum, polysorbate 80, carboxymethylcellulose sodium and like to give an emulsion or a suspension. If desired, corrigents, coloring agents and preservatives may be added to these liquid preparations.

For formulation of injections, the compound of the present invention, if necessary after the addition of pH adjusting agents (e.g., hydrochloric acid, sodium hydroxide, lactic acid, sodium lactate, sodium monohydrogen phosphate and sodium dihydrogen phosphate) or isotonizing agents (e.g., sodium chloride and glucose), is dissolved in distilled water for injection, and the solution is subjected to aseptic filtration and filled into ampules, or further mixed with mannitol, dextrin, cyclodextrin, gelatin and the like, and lyophillized to give an injection which should be dissolved on use. In addition, the compound of the present invention is mixed with lecithin, polysorbate 80, polyoxyethylene hydrogenated caster oil, and the like, and emulsified in water to give an injectional emulsion.

For formulation of rectal administration forms, the compound of the present invention together with bases for suppositories (e.g., tri-, di- or mono-gryceride of cacao fatty acid and polyethylene glycol) is melted under warming, poured into mold and cooled, or the compound of the present invention is dissolved into polyethylene glycol, soy bean oil and the like and then encupsulated with gelatin film.

For formulation of preparations for skin external application, the compound of the present invention may be mixed with white vaseline, beeswax, liquid paraffin, polyethylene glycol and the like, and kneaded, if necessary, under warming to give an ointment, or the mixture is kneaded together with adhesives (e.g., rosin and alkyl acrylate copolymer), and spread on nonwoven fabrics (e.g., polyethylene) to give a tape

preparation.

For formulation of inhalations, the compound of the present invention is dissolved or dispersed in propellants (e.g., flon gas and carbon dioxide gas), and filled into pressure containers to give an aerosol preparation.

The dosage of the compound of the present invention depends on the age, body weight and disease conditions of the patient, but usually it is preferably from about 0.1 mg to 1 g in a single or several divided doses per man per day.

The present invention is illustrated by the following synthesis examples and a test example.

Synthesis Example 1

5'-O-Stearoyl-3'-deoxyinosine (Compound 1: Formula [I] wherein A is a nitrogen atom, B is a carbon atom, R at the 2'-position is a hydrogen atom, Y is a hydrogen atom, R at the 5'-position is stearoyl)

To an anhydrous pyridine solution (15 ml) of 504 mg (2 mmol) of 3'-deoxyinosine was added 1.52 g (5 mmol) of stearoyl chloride, and the reaction was carried out with stirring at room temperature for 6 hours. The reaction solution was poured into 500 ml of ice water, and the formed precipitate was collected by filtration.

The resulting precipitate was crystallized from hot ethanol containing water to give 660 mg (yield: 63.6%) of 5'-O-stearoyl-3'-deoxyinosine.
m.p. 184 - 187 °C.
MS (m/z) 514 ($M^+$)

Elementary analysis for $C_{26}H_{46}N_4O_5$

Calcd. C, 64.84; H, 8.94; N, 10.80

Found C, 64.81; H, 8.92; N, 10.63

Synthesis Example 2

5'-O-Palmitoyl-3'-deoxyinosine (Compound 2: Formula [I] wherein A is a nitrogen atom, B is a carbon atom, Y is a hydrogen atom, R at the 2'-position is a hydrogen atom, and R at the 5'-position is palmitoyl)

To an anhydrous pyridine solution (30 ml) of 756 mg (3 mmol) of 3'-deoxyinosine was added 1.65 g (6 mmol) of palmitoyl chloride, and the reaction was carried out with stirring at room temperature for 5 hours. To the reaction solution was added 5 ml of ice water, and the mixture was stirred for 30 minutes.

The solvent was evaporated under reduced pressure, and then the residue, after ethanol azeotrope, was concentrated to dryness. The residue was purified on silica gel column (2.5 x 30 cm) with 30% ethanol/chloroform as an eluent and then crystallized from hot ethanol containing water to give 537 mg (yield: 37%) of 5'-O-palmitoyl-3'-deoxyinosine.
m.p. 189 - 192 °C.
MS (m/z) 490 ($M^+$)

Synthesis Example 3

5'-O-Decanoyl-3'-deoxyinosine (Compound 3: Formula [I] wherein A is a nitrogen atom, B is a carbon atom, Y is a hydrogen atom, R at the 2'-position is a hydrogen atom, and R at the 5'-position is decanoyl)

To an anhydrous pyridine solution (30 ml) of 756 mg (3 mmol) of 3'-deoxyinosine was added 1.14 g (6 mmol) of decanoyl chloride, and the reaction was carried out with stirring at room temperature for 3 hours.

After the reaction, a treatment similar to that of Synthesis Example 2 and crystallization from methanol gave 539 mg (yield: 44%) of 5'-O-decanoyl-3'-deoxyinosine.
m.p. 170 - 173 °C.
MS (m/z) 406 ($M^+$)

Synthesis Example 4

2',5'-Di-O-stearoyl-3'-deoxyinosine (Compound 4: Formula [I] wherein A is a nitrogen atom, B is a

carbon atom, Y is a hydrogen atom, both R's at the 2'- and 5'-positions are each stearoyl)

To a mixture of anhydrous pyridine (15 ml) and dimethylformamide (10 ml) were added 504 mg (2 mmol) of 3'-deoxyinosine and 20 mg of 4-dimethylaminopyridine.

Then 2.42 g (8 mmol) of stearoyl chloride was added to the solution, and the reaction was carried out with stirring at 70° C for 8 hours.

The reaction solution was poured into 300 ml of ice water and distributed with chloroform, and the resulting chloroform layer was concentrated under reduced pressure. The resulting residue was purified on silica gel column (2.5 x 20 cm) with 4% ethanol/chloroform as an eluent and then crystallized from ethanol to give 1.33 g (yield; 85%) of 2',5'-di-O-stearoyl-3'-deoxyinosine.

m.p. 76 - 78° C.

MS (m/z) 784 ($M^+$)

Synthesis Example 5

5'-O-Thexyldimethylsilyl-3'-deoxyinosine (Compound 5: Formula [I] wherein A is a nitrogen atom, B is a carbon atom, Y is a hydrogen atom, R at the 2'-position is a hydrogen atom, and R at 5'-position is thexyldimethylsilyl)

To a dimethylformamide solution (15 ml) of 756 mg (3 mmol) of 3'-deoxyinosine and 408 mg (6 mmol) of imidazole was added 0.89 ml (4.5 mmol) of thexyldimethylsilyl chloride, and the reaction was carried out with stirring at room temperature for 15 hours. Ethyl acetate and water were added to the reaction solution for distribution, and the organic solvent layer was concentrated under reduced pressure to give 755 mg (yield: 64%) of 5'-O-thexyldimethylsilyl-3'-deoxyinosine as crystals.

m.p. 197 - 199° C.

MS (m/z) 395 $(M + 1)^+$

379 $(M - 15)^+$

Synthesis Example 6

2'-O-Stearoyl-3'-deoxyinosine (Compound 6: Formula [I] wherein A is a nitrogen atom, B is a carbon atom, Y is a hydrogen atom, R at the 2'-position is stearoyl, and R at the 5'-position is a hydrogen atom)

In a mixture of anhydrous pyridine (15 ml) and dimethylformamide (15 ml) were dissolved 798 mg (2 mmol) of 5'-O-thexyldimethylsilyl-3'-deoxyinosine obtained in Synthesis Example 5 and 20 mg of 4-dimethylaminopyridine. To this solution was added 1.21 g (4 mmol) of stearoyl chloride, and the reaction was carried out with stirring at 70° C for 5 hours.

Ethyl acetate and water were added to the reaction solution for distribution, and the organic solvent layer was purified on silica gel column (2.5 x 28 cm) with 2% ethanol/chloroform as an eluent to give 1.30 g (yield: 90%) of 2'-O-stearoyl-5'-O-thexyldimethylsilyl-3'-deoxyinosine.

To a solution of 1.25 g (1.89 mmol) of the above compound in anhydrous tetrahydrofuran (15 ml) were added 2.5 ml of 1 M tetrabutylammonium fluoride and 140 $\mu\ell$ of acetic acid, and the reaction was carried out with stirring at room temperature for 5 hours. The reaction solution was concentrated to dryness to give a residue, which was then purified on silica gel column (2.5 x 23 cm) with 30% ethanol/chloroform as an eluent to give 619 mg (yield: 63%) of 2'-O-stearoyl-3'-deoxyinosine.

m.p. 153 - 156° C.

MS (m/z) 518 ($M^+$)

Synthesis Example 7

5'-O-tert-Butyldimethylsilyl-3'-deoxyinosine (Compound 7: Formula [I] wherein A is a nitrogen atom, B is a carbon atom, Y is a hydrogen atom, R at the 2'-position is a hydrogen atom, and R at the 5'-position is tert-butyldimethylsilyl)

To a dimethylformamide solution (10 ml) of 1.51 g (6 mmol) of 3'-deoxyinosine and 817 mg (12 mmol) of imidazole was added 1.29 g (8.6 mmol) of tert-butyldimethylsilyl chloride, and the reaction was carried out with stirring at room temperature for 2 days.

The reaction solution was poured into 300 ml of ice water and distributed with chloroform, and the chloroform layer was concentrated under reduced pressure. The resulting residue was crystallized from ethyl acetate to give 1.8 g (yield: 82%) of 5'-O-tert-butyldimethylsilyl-3'-deoxyinosine.

m.p. 219 - 220° C.

MS (m/z) 366 ($M^+$)

Synthesis Example 8

2'-O-tert-Butyldimethylsilyl-3'-deoxyinosine (Compound 8: Formula [I] wherein A is a nitrogen atom, B is a carbon atom, Y is a hydrogen atom, R at the 2'-Position is tert-butyldimethylsilyl, and R at the 5'-position is a hydrogen atom)

To a dimethylformamide solution (15 ml) of 756 mg (3 mmol) of 3'-deoxyinosine and 1.23 g (18 mmol) of imidazole was added 1.36 g (9 mmol) of tert-butyldimethylsilyl chloride, and the reaction was carried out with stirring at room temperature for 17 hours. Ice water and ethyl acetate were added to the reaction solution for distribution, the organic solvent layer was concentrated to dryness, and the residue was dissolved in 50 ml of dioxane. To the dioxane solution was added 3 ml of 1N-hydrochloric acid, and the reaction was carried out with stirring at room temperature for 5 hours. The mixture was neutralized by adding 1N-sodium hydroxide solution, and the solvent was evaporated under reduced pressure. The resulting residue was distributed with chloroform and water, and the solvent in the chloroform layer was evaporated under reduced pressure. The residue was crystallized from ethyl acetate/hexane to give 838 mg (yield: 76%) of 2'-O-tert-butyldimethylsilyl-3'-deoxyinosine.

m.p. 178 - 180 °C.

MS (m/z) 367 (M + 1)

Synthesis Example 9

5'-O-Benzyl-3'-deoxyinosine (Compound 9: Formula [I] wherein A is a nitrogen atom, B is a carbon atom, Y is a hydrogen atom, R at the 2'-position is a hydrogen atom, and R at the 5'-position is benzyl)

To an anhydrous tetrahydrofuran solution (15 ml) of 184 mg (0.5 mmol) of 2'-O-tert-butyldimethylsilyl-3'-deoxyinosine obtained in Synthesis Example 8 was added sodium hydride (60%, 60 mg), and stirring was continued until evolution of hydrogen gas ceased. After the reaction, 80 µℓ of benzyl bromide and 222 mg of tetrabutylammonium iodide ($[CH_3(CH_2)_3]_4NI$) were added to the reaction solution, and the reaction was carried out with stirring at room temperature under an argon stream for 20 hours.

The reaction solution was concentrated to dryness under reduced pressure, the residue was dissolved in 50 ml of ethyl acetate and washed with water, and the solvent was evaporated. The residue was purified on silica gel column (3 x 20 cm) with 4% ethanol/chloroform as an eluent to give 144 mg (yield: 63%) of 5'-O-benzyl-2'-O-tert-butyldimethylsilyl-3'-deoxyinosine.

To a solution of the above compound in 5 ml of anhydrous tetrahydrofuran was added 0.5 ml of 1 M tetrabutylammonium fluoride, and the reaction was carried out with stirring at room temperature for 30 minutes, followed by concentration to dryness under reduced pressure. The residue was purified on silica gel column (1.5 x 20 cm) with 20% ethanol/chloroform as an eluent and then crystallized from ethanol/ethyl acetate to give 87 mg (yield: 81%) of 5'-O-benzyl-3'-deoxyinosine.

m.p. 178 - 179 °C.

MS (m/z) 342 ($M^+$)

Synthesis Example 10

5'-O-p-Chlorobenzyl-3'-deoxyinosine (Compound 10: Formula [I] wherein A is a nitrogen atom, B is a carbon atom, Y is a hydrogen atom, R at the 2'-position is a hydrogen atom, and R at the 5'-position is a p-chlorobenzyl)

To an anhydrous tetrahydrofuran solution (20 ml) of 732 mg (2 mmol) of 2'-O-tert-butyldimethylsilyl-3'-deoxyinosine obtained in Synthesis Example 8 was added sodium hydride (60%, 60 mg), and the reaction was carried out with stirring until evolution of hydrogen gas ceased.

After the reaction, 411 mg (2 mmol) of p-chlorobenzyl bromide was added to the reaction solution, and the reaction was carried out with stirring at room temperature for 3 hours. After the reaction, 2.5 ml of 1 M tetrabutylammonium fluoride was added to the reaction solution, the mixture was stirred at room temperature for an hour, and then the reaction solution was concentrated to dryness under reduced pressure. The residue was purified on silica gel column (2.5 x 20 cm) with 20% ethanol/chloroform as an eluent, and crystallized from ethanol to give 228 mg (yield: 30%) of 5'-O-p-chlorobenzyl-3'-deoxyinosine.

Elementary Analysis for $C_{17}H_{17}N_4O_2Cl$

Calcd. C, 54.19; H, 4.55; N, 14.87

Found C, 55.10; H, 4.55; N, 14.73

MS (m/z) 376 ($M^+$)

Synthesis Example 11

1-(β-D-2,5-Di-O-stearoyl-3-deoxyribofuranosyl)allopurinol (Compound 11: Formula [I] wherein A is a carbon atom, B is a nitrogen atom, Y is a hydrogen atom and both R's at the 2'- and 5'-positions are each stearoyl)

To a solution of allopurinol-3'-deoxyriboside (328 mg, 1.3 mmol) in pyridine (13 ml) was added stearoyl chloride (1.3 g, 4.3 mmol), and stirring was continued at room temperature for 24 hours. The reaction solution was poured into ice water (300 ml), and the formed precipitate was collected by filtration, purified on silica gel column with 5% methanol/chloroform as an eluent and recrystallized twice from ethanol to give 784 mg (yield: 77%) of the end compound as powdered crystals.

Elementary Analysis for $C_{46}H_{88}N_4O_6$

Calcd. C, 70.37; H, 10.27; N, 7.14

Found C, 70.09; H, 10.14; N, 6.87

NMR spectrum ($CDCl_3$, ppm)
0.88(6H, t), 1.24-1.26(28H, s),
1.60-1.69(4H, m), 2.18-2.23(1H, m),
2.34-2.39(4H, m), 2.51-2.54(1H, m),
4.26-4.31(1H, m), 4.43-4.46 (1H, m),
4.63-4.65(1H, m), 5.64(1H, d), 6.07(1H, s),
8.02(1H, s), 8.14(1H, s), 12.91(1H, s)

Synthesis Example 12

1-(β-D-2,3,5-Tri-O-stearoylribofuranosyl)allopurinol (Compound 12: Formula [I] wherein A is a carbon atom, B is a nitrogen atom, Y is stearoyloxy, and both R's at the 2'- and 5'-positions are each stearoyl)

Following a similar manner to that of Synthesis Example 11, 1.25 g (yield: 59%) of the end compound was obtained from allopurinol riboside (536 mg, 2 mmol).

Elementary Analysis for $C_{64}H_{114}N_4O_8$

Calcd. C, 70.00; H, 10.76; N, 5.25

Found C, 71.89; H, 10.63; N, 5.08

NMR spectrum ($CDCl_3$, ppm)
0.88(9H, t), 1.25-1.30(42H, s),
1.55-1.65(9H, m), 2.30-2.37(9H, m),
4.20-4.24(1H, m), 4.41-4.45(1H, m),
5.79-5.82(1H, m), 5.90-5.93(1H, m),
6.46(1H, d), 8.00(1H, s), 8.16(1H, s),
11.47(1H, s)

Synthesis Example 13

1-(β-D-5-O-tert-Butyldimethylsilyl-3-deoxyribofuranosyl)allopurinol (Compound 13: Formula [I] wherein A is a carbon atom, B is a nitrogen atom, Y and R at the 2'-position are each a hydrogen atom, and R at the 5'-positions is tert-butyldimethylsilyl)

To a solution of allopurinol-3'-deoxyriboside (504 mg, 2 mmol) in dimethylformamide (DMF) (5 ml) were added imidazol (408 mg, 6 mmol) and tert-butyldimethylsilyl chloride (452 mg, 3 mmol), and stirring was continued at room temperature for 24 hours. The solvent of the reaction solution was evaporated under reduced pressure, the residue was distributed with water and chloroform, and then the organic layer was dried over anhydrous sodium sulfate. The solvent was evaporated, and the resulting residue was recrystallized from ethyl acetate to give 470 mg (yield: 70%) of the end compound as powdered crystals.

Elementary Analysis for $C_{16}H_{26}N_4O_4Si$

Calcd. C, 52.44; H, 7.15; N, 15.29

Found C, 52.35; H, 7.09; N, 15.36

NMR spectrum ($CDCl_3$, ppm)
0.03(3H, s), 0.04(3H, s), 0.91(9H, s),
1.88-1.94(1H, m), 2.19-2.27(1H, m),
3.70-3.75(1H, m), 3.88-3.93(1H, s),
4.37-4.40(1H, m), 4.51(1H, d), 5.74(1H, bs),
5.88(1H, d), 8.06(1H, s), 8.24(1H, s),
12.38(1H, s)

Synthesis Example 14

1-(β-D-5-O-tert-Butyldimethylsilylribofuranosyl)allopurinol (Compound 14: Formula [I] wherein A is a carbon atom, B is a nitrogen atom, Y is a hydroxyl group, R at the 2'-position is a hydrogen atom, and R at the 5'-positions is tert-butyldimethylsilyl)

Following a similar manner to that a Synthesis Example 13, 470 mg (yield: 66%) of the end compound was obtained from allopurinol riboside (536 mg, 2 mmol).
m.p. 140 - 141 °C.

Elementary Analysis for $C_{16}H_{26}N_4O_5Si$

Calcd. C, 50.24; H, 6.85; N, 14.65

Found C, 50.98; H, 6.96; N, 14.48

NMR spectrum ($CDCl_3$, ppm)
0.01(6H, s), 0.82(9H, s), 3.60-3.64(1H, m),
3.72-3.76(1H, m), 3.90-3.94(1H, m),
4.25-4.28(1H, m), 4.53-4.56(1H, m),
5.17(1H, bs), 5.45(1H, bs), 6.08(1H, d),
8.13(1H, s), 8.14(1H, s), 12.31(1H, s)

Test Example 1

Therapeutic Effect on Mice Infected with Leishmania Donovani

Five BALB/c mice were used for each group (control group, saline treated group, the test compound treated group and pentostam treated group), and groups other than control group were inoculated with 1 x $10^8$ of Leishmnia donovani 25-15 M strain.

For the test compound treated group, the test compound (50 mg/kg or 100 mg/kg) suspended in saline was administered orally after 2 hours of the protozoa inoculation, and the oral administration was continued for 12 days. For saline treated group and pentostam treated group, saline and pentostam (40 mg/kg)

suspended in saline, respectively, were administered intramuscularly on days 2, 4, 7, 9 and 11 after inoculation of the protozoa. All mice provided to the test were fasted for 3 hours prior to the administration, and the body weight was measured every day.

Two weeks after the protozoa inoculation, all mice provided to the test were thoracotomized under ether anesthesia according to a similar manner to the known method [Clin. Exp. Immunol., 30, 119 (1977)], the blood was removed, and the liver weight and spleen weight were measured. The stamp smear of the liver prepared according to a usual manner was fixed with methanol and stained with giemsa, and the number of the amastigotes relative to 1000 cell nuclei of the liver stamp smear was counted.

For a monitor of the systemic side effects, the hematocrit and plasma protein content were measured by the microcapillary and the refractometer, respectively.

The therapeutic effect of the test compound to mice infected with Leishmania donovani is shown in Fig. 1. It is demonstrated from Fig. 1 that the number of the amastigotes in mice treated with each of the compounds of the present invention is smaller than that in mice treated with known 3'-deoxyinosine, and the compounds of the present invention inhibit the growth of Leishmania donovani, and then the inhibition effect is comparable to that of pentostam used now as a therapeutic agent.

Figs. 2 to 4 show the results of the measurements of the body weight change, the weight of the liver and the spleen, hematocrit and the plasma protein content. It is apparent from these figures that any side effect other than increase of the spleen weight is not observed.

INDUSTRIAL UTILIZABILITY

The nucleoside derivatives of the present invention not only have more enhanced main effect (antiprotozoal effect) than the prior compound 3'-deoxyinosine but also have reduced side effects comparable to 3'-deoxyinosine, therefore, they are useful for the improvement of the disease conditions caused by protozoa. Especially, there is the effect that the nucleoside derivatives of the present invention are usable even in countries, regions and places without good sanitary conditions because of manifesing the pharmacological effect by oral administration as well.

**Claims**

1. A nucleoside derivative represented by Formula [I]

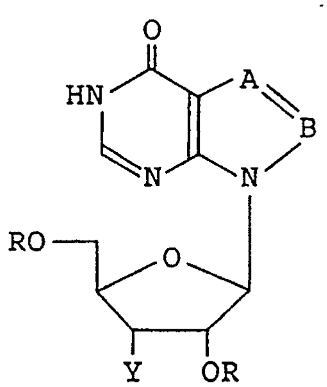

[I]

[wherein A and B are different, and are each a carbon atom or a nitrogen atom, Y is a hydrogen atom, a hydroxyl group or -OR, and R's are the same or different, and are each a hydrogen atom, an aralkyl group, a silyl group or an acyl group, with the exception of the compounds represented by the following combinations:

(1) A is a nitrogen atom, B is a carbon atom, Y is a hydroxyl group or -OR, and R's are the same or different, and are each a hydrogen atom, an aralkyl group, a silyl group or an acyl group,

(2) A is a nitrogen atom, B is a carbon atom, Y is a hydrogen atom, and all R's are hydrogen atoms simultaneously, and

(3) A is a carbon atom, B is a nitrogen atom, Y is a hydroxyl group, and all R's are hydrogen atoms simultaneously], or a pharmaceutically acceptable salt thereof.

2. The nucleoside derivative or the pharmaceutically acceptable salt thereof of Claim 1 which is a

nucleoside derivative represented by Formula [I']

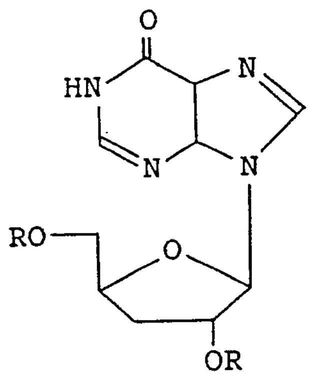

[I']

(wherein R's are the same or different, and are each a hydrogen atom, an aralkyl group, a silyl group or an acyl group, with the exception of the compound in which all R's are hydrogen atoms simultaneously), or a pharmaceutically acceptable salt thereof.

**3.** The nucleoside derivative or the pharmaceutically acceptable salt thereof of Claim 2 which is a nucleoside derivative selected from the group consisting of:
a 5'-O-aralkyl-3'-deoxyinosine;
a 2'-O-aralkyl-3'-deoxyinosine;
a 2',5'-di-O-aralkyl-3'-deoxyinosine;
a 5'-O-silyl-3'-deoxyinosine;
a 2'-O-silyl-3'-deoxyinosine;
a 2',5'-di-O-silyl-3'-deoxyinosine;
a 5'-O-acyl-3'-deoxyinosine;
a 2'-O-acyl-3'-deoxyinosine and
a 2',5'-di-O-acyl-3'-deoxyinosine,
or a pharmaceutically acceptable salt thereof.

**4.** The nucleoside derivative or the pharmaceutically acceptable salt thereof of Claim 2 which is a nucleoside derivative selected from the group consisting of:
5'-O-benzyl-3'-deoxyinosine;
5'-O-stearoyl-3'-deoxyinosine;
2'-O-thexyldimethylsilyl-3'-deoxyinosine and
2'-O-tert-butyldimethylsilyl-3'-deoxyinosine,
or a pharmaceutically acceptable salt thereof.

**5.** The nucleoside derivative or the pharmaceutically acceptable salt thereof of Claim 1 which is a nucleoside derivative represented by Formula [I"]

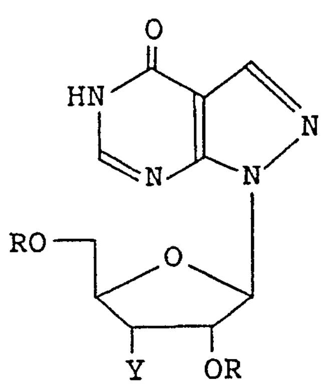

[I"]

(wherein Y is a hydrogen atom, a hydroxyl group or -OR, and R's are the same or different, and are

each a hydrogen atom, an aralkyl group, a silyl group or an acyl group, with the exception of the compound in which Y is a hydroxyl group and all R's are hydrogen atoms simultaneously), or a pharmaceutically acceptable salt thereof.

6. The nucleoside derivative or the pharmaceutically acceptable salt thereof of Claim 5 which is a nucleoside derivative selected from the group consisting of:
   a 1-(β-D-2,5-di-O-acyl-3-deoxyribofuranosyl)allopurinol;
   a 1-(β-D-2,3,5-tri-O-acylribofuranosyl)allopurinol;
   a 1-(β-D-5-O-acyl-3-deoxyribofuranosyl)allopurinol;
   a 1-(β-D-5-O-acylribofuranosyl)allopurinol;
   a 1-(β-D-5-O-aralkyl-3-deoxyribofuranosyl)allopurinol;
   a 1-(β-D-5-O-aralkylribofuranosyl)allopurinol;
   a 1-(β-D-2-O-silylribofuranosyl)allopurinol;
   a 1-(β-D-2-O-silyl-3-deoxyribofuranosyl)allopurinol;
   a 1-(β-D-3-deoxyribofuranosyl)allopurinol and
   a 1-(β-D-5-O-silyl-3-deoxyribofuranosyl)allopurinol,
   or a pharmaceutically acceptable salt thereof.

7. An antiprotozoal agent which comprises containing as an effective ingredient the nucleoside derivative or the pharmaceutically acceptable salt thereof of Claim 1.

8. The antiprotozoal agent of Claim 7 which is an oral administration preparation.

FIG. 1

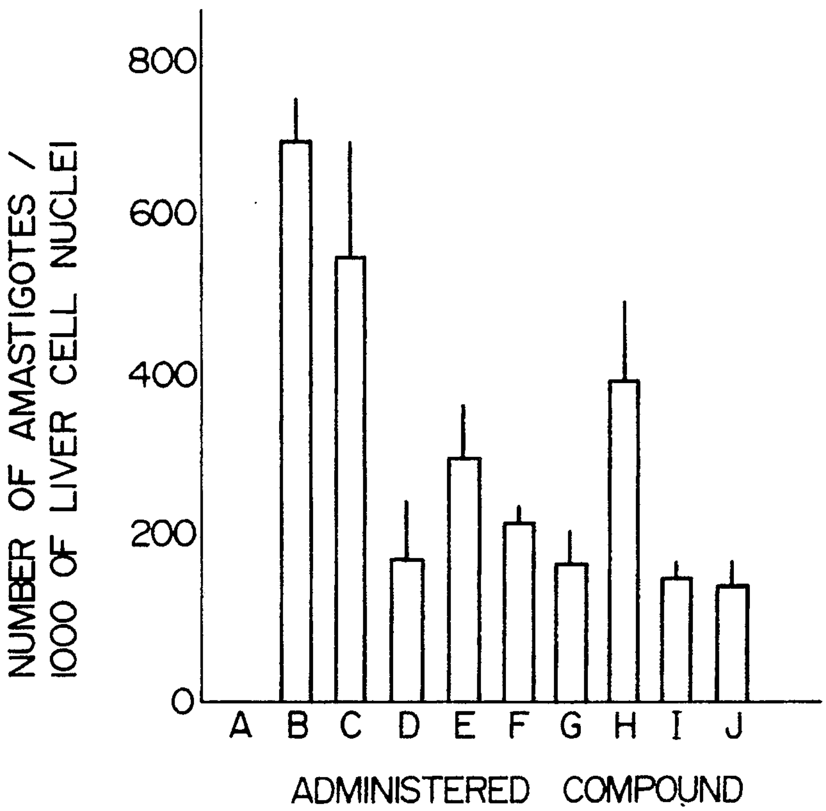


A : CONTROL
B : SALINE
C : 3'-DEOXYINOSINE (100mg/kg)
D : COMPOUND 9 (100mg/kg)
E : COMPOUND 10 (100mg/kg)
F : COMPOUND 7 (100mg/kg)
G : COMPOUND 8 (100mg/kg)
H : COMPOUND 1 (50mg/kg)
I : COMPOUND 1 (100mg/kg)
J : PENTOSTAM (40mg/kg)

FIG. 2

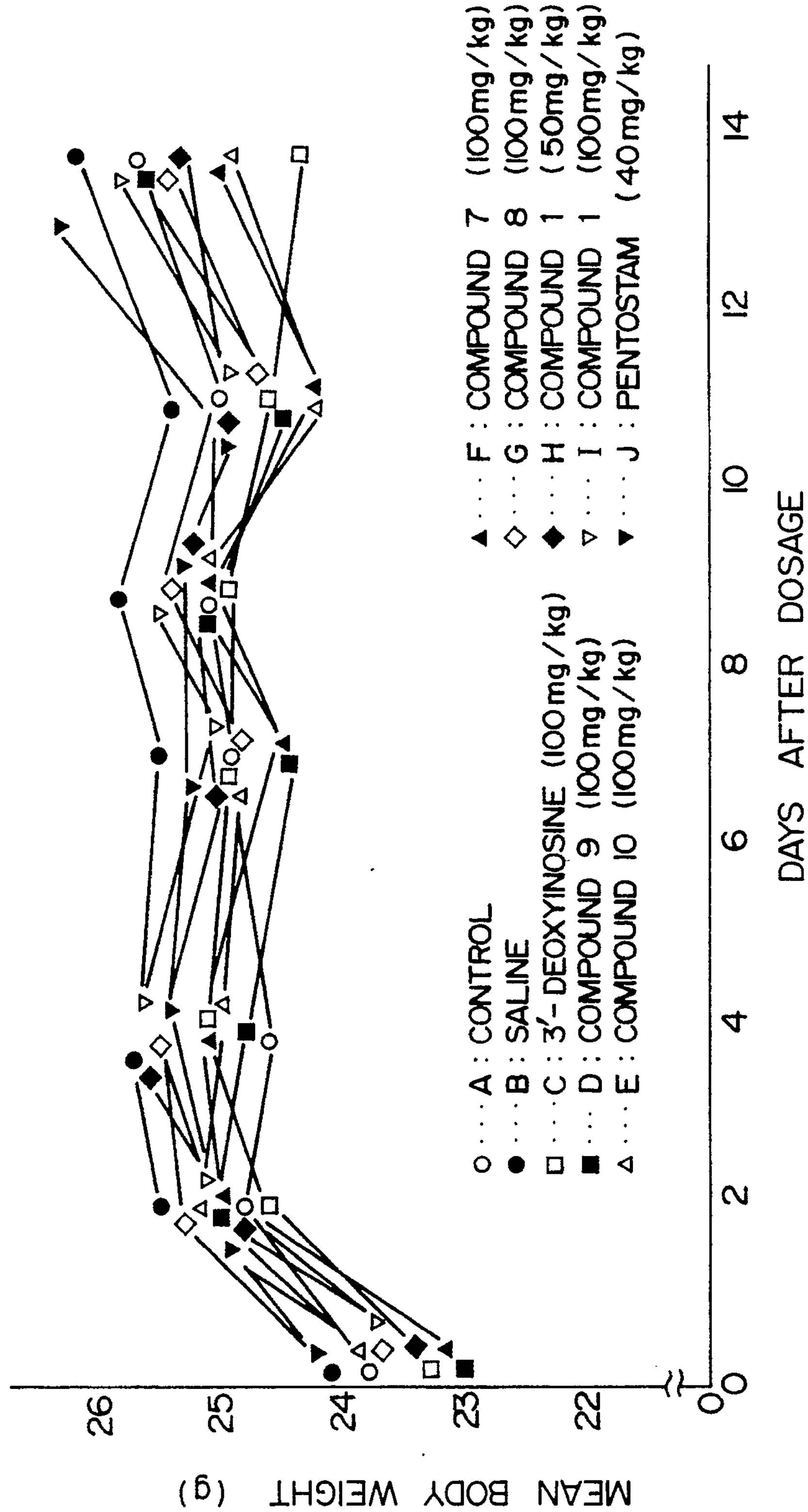
MEAN BODY WEIGHT (g)
DAYS AFTER DOSAGE
0
2
4
6
8
10
12
14
22
23
24
25
26
○ ····· A : CONTROL
● ····· B : SALINE
□ ····· C : 3'-DEOXYINOSINE (100mg/kg)
■ ····· D : COMPOUND 9 (100mg/kg)
△ ····· E : COMPOUND 10 (100mg/kg)
▲ ····· F : COMPOUND 7 (100mg/kg)
◇ ····· G : COMPOUND 8 (100mg/kg)
◆ ····· H : COMPOUND 1 (50mg/kg)
▽ ····· I : COMPOUND 1 (100mg/kg)
▼ ····· J : PENTOSTAM (40mg/kg)

FIG. 3

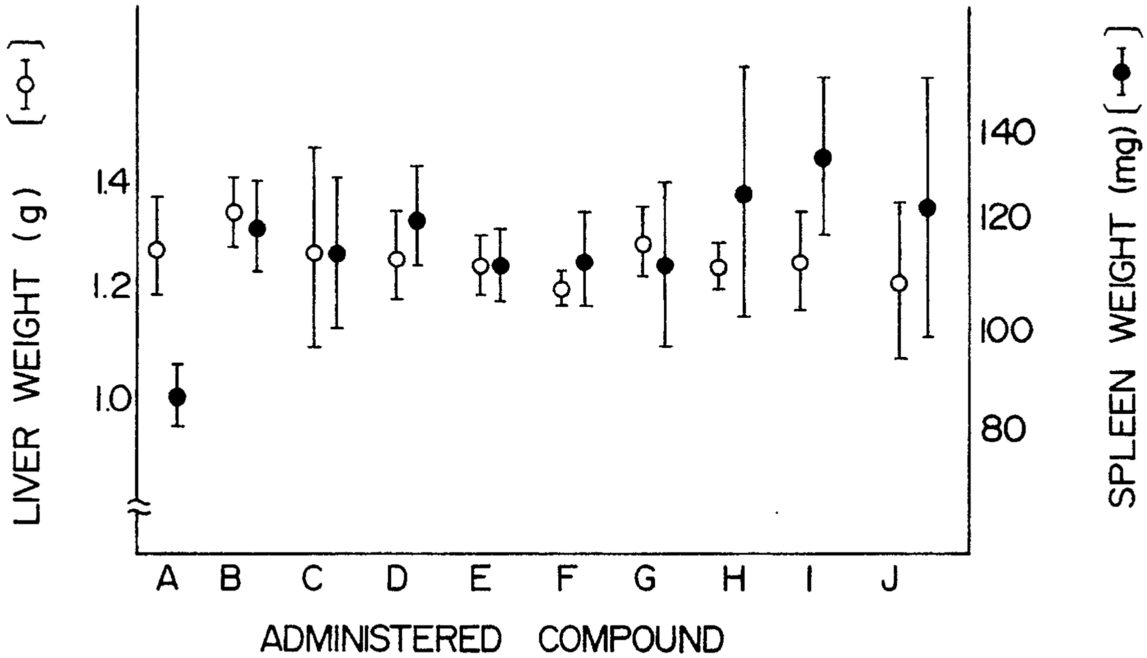


A : CONTROL
B : SALINE
C : 3'-DEOXYINOSINE (100mg/kg)
D : COMPOUND 9 (100mg/kg)
E : COMPOUND 10 (100mg/kg)
F : COMPOUND 7 (100mg/kg)
G : COMPOUND 8 (100mg/kg)
H : COMPOUND 1 (50mg/kg)
I : COMPOUND 1 (100mg/kg)
J : PENTOSTAM (40mg/kg)

FIG. 4

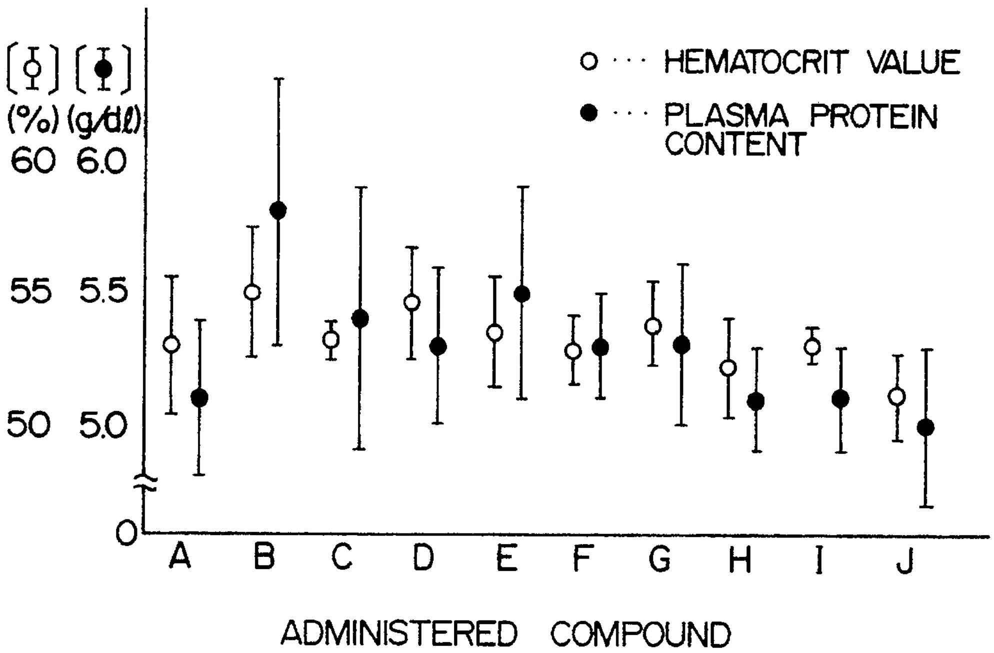


A : CONTROL
B : SALINE
C : 3'-DEOXYINOSINE (100mg/kg)
D : COMPOUND 9 (100mg/kg)
E : COMPOUND 10 (100mg/kg)
F : COMPOUND 7 (100mg/kg)
G : COMPOUND 8 (100mg/kg)
H : COMPOUND 1 (50mg/kg)
I : COMPOUND 1 (100mg/kg)
J : PENTOSTAM (40mg/kg)

# INTERNATIONAL SEARCH REPORT

International Application No PCT/JP90/01371

**I. CLASSIFICATION OF SUBJECT MATTER** (if several classification symbols apply, indicate all) [6]

According to International Patent Classification (IPC) or to both National Classification and IPC

Int. $Cl^5$ C07H19/16, C07H19/23, A61K31/70

**II. FIELDS SEARCHED**

Minimum Documentation Searched [7]

| Classification System | Classification Symbols |
|---|---|
| IPC | C07H19/16, 167, 19/23, A61K31/70, 71 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| X | JP, A, 60-184018 (Takeda Chemical Industries, Ltd.), September 19, 1985 (19. 09. 85) | 1-4, 7, 8 |
| X | Chemische Berichte, Vol. 114, No. 5, p. 1610-1623 (1981), the compounds represented by the formulae 9a and 9b | 1, 5, 6 |
| X | J. Medicinal Chemistry, Vol. 26, No. 11, p. 1601-1606 (1983), the compound represented by the formula 9, refer to p. 1604-1605 | 1, 5, 6 |
| Y | J. Medicinal Chemistry, Vol. 25, No. 9, p. 1040-1044 (1982), the compound No. 1 | 1, 5-8 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| January 8, 1991 (08. 01. 91) | January 21, 1991 (21. 01. 91) |
| International Searching Authority | Signature of Authorized Officer |
| Japanese Patent Office | |